# EUROPEAN PATENT APPLICATION

(11) **EP 0 534 916 A1**
(43) Date of publication of application: **31.03.1993**
(21) Application number: 92830501.0
(22) Date of filing: 17.09.1992
(51) Int. Cl.: A61K 9/127, A61K 31/485, A61K 9/00

(54) **The use of liposomal formulations containing papaverine for the treatment of male impotence**

(30) Priority: 20.09.1991 IT MI912511
(71) Applicant: RICERCHE DI SCHIENA S.N.C. DEL DR. MICHELE G. DI SCHIENA & C., I-20080 Cisliano (Milano) (IT)
(72) Inventor: Di Schiena, Michele Giuseppe, I-20080 Cisliano (Milano) (IT)
(74) Representative: Minoja, Fabrizio

(57) **Abstract**

Liposomal compositions containing papaverine, useful in the diagnosis and therapy of male sexual impotence, and a process for the preparation thereof are herein described.

## Description

The present invention relates to topical liposomal compositions containing a papaverine hydrosoluble salt, said compositions being useful in the diagnosis and therapy of male sexual impotence due to insufficient erectile capacity of penis consequent to vascular alterations of the organ.

Male sexual impotence is a worldwide pathology and is continuously increasing especially in the wealthy countries, thus establishing a severe social and health problem.

Among the various forms of male sexual impotence the most common one is due to insufficient penis erection subsequent to vascular alterations, and depends from many factors, most of them are originated by the modern way of life, such as anxiety, stress, overfeeding, excessive use of voluptuary substances, iatrogenic intoxications, etc.

Since a long time, papaverine, preferably in the form of the hydrosoluble hydrochloride salt, has been successfully used for this kind of impotence, both for diagnostic and for therapeutic use. This compound is directly injected in penis corpora cavernosa wherein, with an up to now unclear mechanism, it allows arterial blood flow and contemporaneously hinders venous blood drain; therefore normal vascular functionality of the penis, which recovers its normal erectile capacity, is restored.

When used for diagnosis, this technique allows the confirmation of impotence vascular etiology, while, when used for therapy, this technique provides a temporary remedy to obtain a proper penis erection, thus allowing the sexual intercourse, further it can be an useful rehabilitation for the male organ.

Notwithstanding the efficacy of said technique, its use requires the strict complying of the instructions, as a misuse could give rise to severe damages to penis functionality and morphology, such as for example, formation of fibrous plaques in the corpora cavernosa, said formation being itself an obstacle to the normal erection. In fact, this technique is seldom accepted by the patient both for psychic and practical reasons, since he must inject the drug by himself, in expectation of the sexual intercourse. Therefore, this technique did not spread as expected.

In order to overcome the risks and drawbacks of the above-described technique, several topical formulations containing papaverine have been proposed, but up to now, the problem has not been solved, as papaverine is not absorbed through the transdermal route.

The present invention surprisingly overcomes the difficulties of the prior art with the use of topical liposomal compositions containing a papaverine hydrosoluble salt to be applied in the penis area.

A preferred embodiment of the present invention provides the use of the papaverine hydrochloride salt.

Papaverine in the liposomal form is capable of crossing the various tissue layers of penis arriving at the corpora cavernosa, where the drug is released in order to exert its pharmacological activity on the vascular district of the organ. By the present invention, the patient complies with the method and avoids risks and drawbacks of the injection method.

Liposomal formulations according to the present invention can be obtained with conventional methods, well-known to the expert technician, for example Deamer's method, Szoka's method and osmotic method may be used.

Liposome size is not critical for the purposes of the present invention; as also the starting materials for liposomes aren't, except they are physiologically acceptable.

Preferably, the compositions of the present invention can be obtained by using a commercial product known as NATIPIDE®II (by Natterman Phospholipid GMBH). This compound is a preformed liposome, consisting of a soybean phospholipid mixture, which has the property to instantaneously liposomize a papaverine hydrochloride solution by simple high speed stirring.

The so obtained composition is in form of a gel, which is appropriately used for the purposes of the present invention.

However, the compositions of the present invention can be suitably prepared by using all those excipients (gelifying, thickening agents, preservatives, perfumes, etc.) known in the art, provided that they are compatible with the liposome structure.

In the compositions of the invention, papaverine concentration may range from 0.1 to 5%, preferably from 1 to 3%.

Both single and daily dosages will be established by the physician, for example from 10 to 30 mg/application.

The compositions of the invention are absorbed by hand massage on the penis area, preferably on the glans, since this area lacks the corneous layer, therefore it allows the active ingredient to penetrate easier.

Preferred examples of topical compositions are gels, creams, emulsions, lotions.

The following example further illustrates the invention.

### EXAMPLE

### Liposomal composition containing 1% papaverine hydrochloride

NATIPIDE®II, having the following composition, was used:

| | |
|---|---|
| Soy phospholipids | 20% ca. |
| Ethanol | 16% ca. |
| Water q.s. to | 100% |
| The following solution was prepared: | |
| Papaverine hydrochloride | 1 g |
| Ethanol | 1.44 g |
| Distilled water | 7.56 g |

The solution was cautiously warmed to 50-60°C, then 90 g of NATIPIDE®II were added and the mixture was homogenized in a high speed mixer (Braun Multi Mix MX32) for 5 minutes.

A yellowish semifluid gel was obtained with the following physico-chemical characteristics:
Particle size (mean) 145 nm
pH: 4.7 (20°C)
Appearance: fluid dispersion without crystals.

This product can directly be used, for example applying 1 g (equal to 10 mg of papaverine hydrochloride) on the glans and absorbing it by hand massage.

Similarly, by using 2 or 3 g of papaverine hydrochloride, liposomes containing 2 or 3% of papaverine hydrochloride were obtained.

The so obtained liposomes can be used as "base" for compositions in the form of gels, creams, lotions, etc., by adding suitable excipients.

## Claims

1. Topical liposomal compositions containing a papaverine hydrosoluble salt.

2. Liposomal compositions according to claim 1, wherein papaverine hydrochloride is the papaverine hydrosoluble salt.

3. Liposomal compositions according to claims 1 and 2, wherein the papaverine hydrosoluble salt is contained in concentrations ranging from 0.1 to 5%.

4. Liposomal compositions according to claims 1 and 2, wherein the papaverine hydrosoluble salt is contained in concentrations ranging from 1 to 3%.

5. Liposomal compositions according to claims 1 to 4 in the form of gels, creams, lotions.

6. The use of liposomal compositions according to claims 1 to 5 for the preparation of a medicament useful for the treatment of male sexual impotence.

7. A process for the preparation of liposomal compositions according to claims 1 to 5, characterized in that an aqueous-alcoholic papaverine hydrochloride solution is homogenized with NATIPIDE®II.
